# EUROPEAN PATENT SPECIFICATION

(11) **EP 3 386 503 B1**
(45) Date of publication and mention of the grant of the patent: **22.06.2022**
(21) Application number: 16874049.6
(22) Date of filing: 12.12.2016
(51) Int. Cl.: A61L 29/16, A61K 9/00, A61K 9/107, A61K 9/08, A61K 9/127, A61K 9/19, A61K 31/404, A61K 31/4418, A61P 9/00

(54) **KITS FOR TREATING VASCULAR DISEASE**
SETS ZUR BEHANDLUNG VON GEFÄSSERKRANKUNGEN
KITS POUR LE TRAITEMENT D'UNE MALADIE VASCULAIRE

(30) Priority: 10.12.2015 US 201562265676 P
(43) Date of publication of application: 17.10.2018
(73) Proprietor: Endoperfusion Solutions, LLC, Atlanta, Georgia 30324 (US)
(72) Inventor: SINGH, Jaipal, Carmel, Indiana 46032 (US)
(74) Representative: Cabinet Laurent & Charras
(86) International application number: PCT/US2016/066167
(87) International publication number: WO 2017/100767

(56) References cited:
- EP-A1- 2 380 604
- CA-A1- 2 595 485
- US-A1- 2004 122 077
- US-A1- 2006 235 070
- US-A1- 2009 258 049
- US-A1- 2010 143 453
- US-A1- 2013 098 357
- US-A1- 2013 156 850
- US-B2- 8 715 230

## Description

This application claims benefit of U.S. Provisional Application No. 62/265,676, filed December 10, 2015.

### TECHNICAL FIELD

This application relates generally to kits for treating vascular diseases, including peripheral vascular disease (PVD).

### BACKGROUND

The high morbidity and mortality of patients with peripheral vascular disease (PVD) is a major economic healthcare burden worldwide. Currently, pharmacological agents to effectively treat PVD patients are not available. Use of interventional devices such as stents has produced encouraging early results. Percutaneous balloon angioplasty of the superficial femoral artery and popliteal lesions can also acutely restore blood flow. However, the restenosis rate following balloon angioplasty is extremely high. About 40- 60% of patients require another intervention within a year.

For coronary artery disease, the drug eluting stents (DES) are now common choice. DES have been highly effective in inhibiting neointimal hyperplasia and restenosis following percutaneous angioplasty. However, certain safety concerns with DES still remain. Late stent thrombosis (ST) presumably resulting from delayed endothelial healing and dysfunctional endothelial function, vascular inflammation remains an issue. Prolonged use of dual antiplatelet therapy (DAPT) in DES patients is associated with significant bleeding complications. In addition, DES are not suitable for many applications, including treating vascular disease in diabetics, treating complex coronary anatomy, including small vessels, treating diffuse disease, and treating bifurcation lesions. More importantly, unlike the efficacy of drug eluting stent in the coronary arteries, DES has failed to show clear superiority in the femoropopliteal arteries compared with uncoated stents. The superficial femoral artery repetitively undergoes high mechanical forces in multiple directions due to flexion, bending, torsion, axial elongation, and shortening. High restenosis and stent fracture are common because of the anatomic complexity of femoral artery. The hypersensitivity and late thrombosis observed in DES in coronary arteries may be exacerbated in peripheral arteries by the mechanical forces and perhaps vascular biological differences between these two vascular beds. Hypersensitivity reactions to polymers in DES is known to produce inflammatory response and late thrombosis. The deficient re-endotheliazation on DES implanted vessels may also contribute to late thrombosis. DES also appear to accelerate neointimal atherosclerosis. Thus DES technology has not been as successful in the treatment of PVD as in the case of coronary artery disease.

Some improvement in restenosis rates have been observed with nitinol stents; however, the restenosis rate remains as high. More recently, a drug-coated balloon (DCB) technology has shown promise for the treatment of coronary in-stent restenosis and peripheral arterial lesions. The drug coated balloon technology is expected to overcome some of the limitations of drug-eluting stents. For example, the technology may reduce stent thrombosis and prolonged use of dual antiplatelet therapy. It may also be applicable in complex disease states such as small vessels and diffuse lesions.

In the current DCB format, paclitaxel is the only drug that has shown promise of efficacy in PVD. Pacletaxel is a microtubule binding drug that indiscriminately inhibits replication of various cell types. Pacletaxel inhibits both smooth muscle and endothelial cells and therefore retards vascular healing following the procedure. Because of slow endothelial healing, it requires longer DAPT treatment which increases risk of bleeding and the cost of treatment. The paclitaxel dose response window for the inhibition of smooth muscle cells is very narrow. At high concentrations the drug becomes toxic to smooth muscle cells and endothelial cells. The drug is highly hydrophobic and not easily amenable to aqueous formulation.

Pacletaxel also produces systemic toxicity. The frequent side effect of paclitaxel treatment in patients is peripheral neuropathy, slow heart rate, uneven heartbeats, seizure, pale skin, easy bruising or bleeding, unusual weakness, fever, chills, body aches, flu symptoms, white patches or sores inside your mouth or on your lips, numbness, tingling, or burning pain in your hands or feet, increased blood pressure (severe headache, blurred vision, buzzing in your ears, anxiety, confusion, chest pain, shortness of breath, joint or muscle pain, mild nausea, vomiting, and diarrhea. The release of drug in the circulation, and long term retention in other organs therefore has the potential of causing these adverse effects. Thus drugs with lower systemic toxicity would be more suitable.

US 2009/258049 A1 discloses a balloon catheter coated with a sol-gel matrix comprising cerivastatin.

### SUMMARY

Methods for treating vascular diseases in a subject in need thereof are disclosed but are not falling under the scope of the claims.

Methods for treating vascular diseases can involve acutely administering a statin locally to a diseased segment of a blood vessel in the subject. The present invention is directed to a kit as defined in claim 1. In the present disclosure "statin" means a statin comprising cerivastatin, or a pharmaceutically acceptable salt, prodrug, clathrate, or solvate thereof.

The statin can be administered in an effective amount to reduce smooth muscle cell proliferation and/or inflammation in the diseased segment of the blood vessel in the subject For example, the statin can be administered in an effective amount to induce DDAH2 gene expression in vascular endothelial cells in the diseased segment of the blood vessel in the subject. The statin can also be administered in an effective amount to decrease CCR2 gene expression in vascular endothelial cells in the diseased segment of the blood vessel in the subject.

The disclosed methods involve local administration of the statin to a diseased segment of a blood vessel in the subject. In some embodiments, the statin can be administered at a dosage of from 1 ng/mm diseased segment of the blood vessel to 10 µg/mm diseased segment of the blood vessel, such as from 10 ng/mm diseased segment of the blood vessel to 1 µg/mm diseased segment of the blood vessel. The disclosed methods can also involve acute administration of the statin. For example, in some embodiments, at least 80% of a dosage of the statin can be delivered to the diseased segment within 5 minutes. In some cases, at least 90% of the dosage of the statin can be delivered to the diseased segment within 1 minute.

Examples of vascular diseases that can be treated include, but are not limited to, atherosclerosis, transplant atherosclerosis, vein-graft atherosclerosis, thrombosis, restenosis, stent restenosis, and angioplasty restenosis.

Methods can involve administering a statin locally to a diseased segment of a blood vessel in the subject by any suitable percutaneous delivery method. In particular, the statin can be delivered using a transluminal catheter, such as a balloon catheter. For example, the statin can be delivered by a drug-coated catheter balloon, a drug-eluting catheter balloon, or a combination thereof. In some cases, the statin is administered by an injection feature affixed to the surface of a catheter balloon. In certain embodiments, the statin can be administered to a diseased segment of a blood vessel using a porous balloon catheter (also referred to as a weeping balloon catheter).

The embodiments according to the invention comprise lipid-based particles (e.g., liposomes, lipid micelles, or a combination thereof). The statin is encapsulated in the lipid-based particles. In certain embodiments, the lipid-based particles can have an average particle size of from 1 nm to 200 nm (e.g., from 5 nm to 100 nm, from 100 nm to 150 nm, or from 5 nm to 50 nm), as determined by dynamic light scattering.

The embodiments according to the invention comprise catheter balloons configured for delivery of a statin locally and acutely to a diseased vascular segment. For example, in some cases, the catheter balloon is completely or partially coated with a statin, wherein a therapeutically effective amount of the statin is released and/or bioavailable of when the balloon is inflated intraluminally.

In an embodiment, the kit according to claim 1 is for use in treating vascular diseases in a subject. In certain embodiments, the drug-eluting balloon catheter comprises a porous balloon catheter.

The lipid-based particles can have an average particle size of from 1 nm to 200 nm (e.g., from 5 nm to 50 nm, or from 100 nm to 150 nm), as determined by dynamic light scattering.

### BRIEF DESCRIPTION OF THE DRAWINGS

Figure 1A is a planar view of an example balloon catheter coated with a statin.
Figure 1B is a cross-sectional view of an example balloon catheter coated with a statin taken along lines A-A in Figure 1A.
Figure 2 is a schematic illustration of an example drug-eluting catheter.
Figure 3 is a transmission electron microscopy (TEM) micrograph illustrating a population of cerivastatin-loaded liposomes.
Figure 4 is a plot of the particle size distribution of cerivastatin-loaded liposomes prepared using the methods described herein.
Figure 5 is a plot of the amount of cerivastatin (in ng) released from cerivastatin-loaded liposomes as a function of time (in hours).
Figure 6 is a plot showing the inhibition of smooth muscle cells (SMCs) by cerivastatin.
Figure 7A is a plot of the proliferation of SMCs at 48 hours following exposure to varying concentrations of free cerivastatin (black bars) and cerivastatin-loaded liposomes (grey bars).
Figure 7B is a plot of the viability of SMCs at 48 hours following exposure to varying concentrations of free cerivastatin (black bars) and cerivastatin-loaded liposomes (grey bars).
Figure 8 is a plot of the concentration of cerivastatin (in ng) remaining within the swine artery segments following intraluminal delivery as a function of time (in days).

### DETAILED DESCRIPTION

### Definitions

As used herein, a "vascular disease" refers to a disease, disorder, or injury of blood vessels, primarily arteries and veins, which transport blood to and from the heart, brain and peripheral organs such as, without limitation, the arms, legs, kidneys and liver. In particular, "vascular disease" can refer to diseases of the coronary arterial and venous systems, the carotid arterial and venous systems, the aortic arterial and venous systems, and the peripheral arterial and venous systems. The disease that may be treated is any that is amenable to treatment with a therapeutic agent, either as the sole treatment protocol or as an adjunct to other procedures such as surgical intervention. The disease may be, without limitation, vascular lesion, atherosclerosis, vulnerable plaque, restenosis, or peripheral arterial disease. Peripheral vascular disease includes arterial and venous diseases of the renal, iliac, femoral, popliteal, tibial and other vascular regions.

Peripheral vascular diseases are generally caused by structural changes in blood vessels caused by such conditions as inflammation and tissue damage. A subset of peripheral vascular disease is peripheral artery disease (PAD). PAD is a condition that is similar to carotid and coronary artery disease in that it is caused by the buildup of fatty deposits on the lining or intima of the artery walls. Just as blockage of the carotid artery restricts blood flow to the brain and blockage of the coronary artery restricts blood flow to the heart, blockage of the peripheral arteries can lead to restricted blood flow to the kidneys, stomach, arms, legs and feet. In particular at present a peripheral vascular disease often refers to a vascular disease of the superficial femoral artery.

As used herein, a "vascular lesion" refers to a vascular disease involving localized pathological change in the vasculature, in particular a change that results in compromising the patency of the vasculature in the vicinity of the lesion. Examples of vascular lesions include, without limitation, saphenous vein graft lesions, de novo lesions, small vessel lesions, restenotic lesions, bifurcation lesions, ostial lesions, left main lesions, chronic total occlusions and occlusions associated with AMI (Acute Myocardial Infarction), STEMI (ST Segment Elevation Myocardial Infarction) or non-STEMI (non-ST Segment Elevation Myocardial Infarction).

"Atherosclerosis" refers to the depositing of fatty substances, cholesterol, cellular waste products, calcium and fibrin on the inner lining or intima of an artery. Smooth muscle cell proliferation and lipid accumulation accompany the deposition process. In addition, inflammatory substances that tend to migrate to atherosclerotic regions of an artery are thought to exacerbate the condition. The result of the accumulation of substances on the intima is the formation of fibrous (atheromatous) plaques that occlude the lumen of the artery, a process called stenosis. When the stenosis becomes severe enough, the blood supply to the organ supplied by the particular artery is depleted resulting in a stroke, if the afflicted artery is a carotid artery, heart attack if the artery is coronary, or loss of organ or limb function if the artery is peripheral.

"Restenosis" refers to the re-narrowing of an artery at or near the site where angioplasty or another surgical procedure was previously performed to remove a stenosis. It is generally due to smooth muscle cell proliferation and, at times, is accompanied by thrombosis. Prior to the advent of implantable stents to maintain the patency of vessels opened by angioplasty, restenosis occurred in 40 - 50% of patients within 3 to 6 months of undergoing the procedure. Post-angioplasty restenosis before stents was due primarily to smooth muscle cell proliferation. However, there were also issues of acute re-closure due to vasospasm, dissection, and thrombosis at the site of the procedure. Stents eliminated acute closure from vasospasm and greatly reduced complications from dissections. The use of IIb/IIIa anti-platelet drugs such as abciximab and epifabatide, and anti-platelet agents such as ticlopidine and clopidogrel, which are anti-thrombotic, reduced the occurrence of post-procedure clotting. Stent placement sites are also susceptible to restenosis due to abnormal tissue growth at the site of implantation. This form of restenosis (referred to as stent restenosis or in-stent restenosis) tends also to occur at 3 to 6 months after stent placement but it is not affected by the use of anti-clotting drugs. This appears to be especially so with diabetic patients. Thus, alternative therapies are continuously being sought to mitigate, preferably eliminate, this type of restenosis, in particular for diabetic patients.

"Vulnerable plaque" refers to an atheromatous plaque that has the potential of causing a thrombotic event and is usually characterized by a thin fibrous cap separating a lipid filled atheroma from the lumen of an artery. The thinness of the cap renders the plaque susceptible to rupture. When the plaque ruptures, the inner core of usually lipid-rich plaque is exposed to blood. This releases tissue factor and lipid components with the potential of causing a potentially fatal thrombotic event through adhesion and activation of platelets and plasma proteins to components of the exposed plaque. The phenomenon of vulnerable plaques has created new challenges in recent years for the treatment of heart disease. Unlike occlusive plaques that impede blood flow, vulnerable plaque develops within the arterial walls, and in its early stages does so without the characteristic substantial narrowing of the arterial lumen which produces symptoms. As such, conventional methods for detecting heart disease, such as an angiogram, may not detect vulnerable plaque growth into the arterial wall.

"Pharmaceutically acceptable", as used herein, refers to compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio, in accordance with the guidelines of agencies such as the Food and Drug Administration.

"Acute administration", as used herein, refers to administration of a pharmaceutical formulation over a short period of time, to deliver a therapeutically effective amount of a statin in a small number of dose administrations, such as, for example, a single dose. Acute administration can thus be distinguished from, for example, extended release of an active agent from an implant over a period of weeks, months, or years.

The term "subject" refers to any individual who is the target of administration or treatment. The subject can be a vertebrate, for example, a mammal. Thus, the subject can be a human or veterinary patient. The term "patient" refers to a subject under the treatment of a clinician, e.g., physician.

As used herein, "treating" refers to the administration of a therapeutically effective amount of a therapeutic agent (e.g., a statin) to a subject known or suspected to be afflicted with a vascular disease.

A "therapeutically effective amount" refers to that amount of a therapeutic agent that will have a beneficial effect, which may be curative or palliative, on the health and well-being of the subject with regard to the vascular disease with which the subject is known or suspected to be afflicted. The beneficial effect on the health and well-being of a subject can include, but it not limited to: (1) curing the vascular disease; (2) slowing the progress of the vascular disease; (3) causing the vascular disease to retrogress; or, (4) alleviating one or more symptoms of the vascular disease. The beneficial effect on the health and well-being of a subject can also include prophylactic outcomes, including but not limited to: (1) preventing or delaying on-set of the vascular disease in the first place; (2) maintaining a vascular disease at a retrogressed level once such level has been achieved by a therapeutically effective amount of a substance; or, (3) preventing or delaying recurrence of the vascular disease after a course of treatment.

### Methods

Devices and methods for treating a vascular disease in a subject are disclosed. Methods of treatment are not claimed. The disclosed devices and methods can be used to treat any disease, disorder, or injury of blood vessels, such as those involving endothelial dysfunction. In some cases, the devices and methods can be used to treat a vascular disease involving reduced nitric oxide (NO) bioavailability. Non-limiting examples of vascular diseases that can be treated include atherosclerosis, transplant atherosclerosis, vein-graft atherosclerosis, thrombosis, restenosis, stent restenosis, and angioplasty restenosis.

In certain embodiments, the vascular disease can comprise stent restenosis. In some cases, the stent restenosis can be restenosis associated with a stent implanted in a coronary artery. In some cases, the stent restenosis can be restenosis associated with a stent implanted in a peripheral artery. In some embodiments, the stent can be a bare metal stent (e.g., a nitinol stent). In some embodiments, the stent can be a drug-eluting stent (e.g., a limus- or paclitaxel-eluting stent).

The vascular disease can be superficial femoral artery disease, optionally in combination with a stent, such as a bare metal stent. The vascular disease can be femoropopliteal artery disease, optionally in combination with a stent, such as a bare metal stent. The vascular disease can be intrapopliteal artery disease, optionally in combination with a stent, such as a bare metal stent. The vascular disease can be intracranial atherosclerotic disease, optionally in combination with a stent, such as a bare metal stent. The vascular disease can be myocardial infarction. The vascular disease can be a vascular lesion, such as de novo coronary artery lesion (optionally in combination with a stent, such as a bare metal stent), bifurcation lesion (optionally in combination with a stent, such as a bare metal stent or T-stent), long and complex lesion, or chronic total occlusion (CTO).

In some embodiments, the vascular disease can be vascular disease in small coronary arteries. In some embodiments, the vascular disease can be peripheral artery disease (PAD), optionally in combination with a stent, such as a bare metal stent (e.g., nitinol stent). In certain embodiments, the vascular disease can be PAD in a diabetic patient. Dual antiplatelet therapy (DAPT), i.e., means the combination of aspirin and a P2Y₁₂ platelet receptor inhibitor, has to be established for a prolonged time after stent implantation in order to avoid potentially catastrophic stent thrombosis (ST). The disclosed devices and methods can also be used to reduce DAPT use following stent implantation. The disclosed devices and methods can also be used to promote rapid healing of treated arteries.

These methods can involve acutely administering a statin locally to a diseased segment of a blood vessel in the subject. For example, the statin can be administered at a dosage of from 1 ng/mm diseased segment of the blood vessel to 10 µg/mm diseased segment of the blood vessel. In some cases, the statin is administered at a dosage from 10 ng/mm diseased segment of the blood vessel to 1 µg/mm diseased segment of the blood vessel. For example, the statin can be administered at a dosage of about 1 ng/mm, 2 ng/mm, 3 ng/mm, 3 ng/mm, 5 ng/mm, 6 ng/mm, 7 ng/mm, 8 ng/mm, 9 ng/mm, 10 ng/mm, 20 ng/mm, 30 ng/mm, 40 ng/mm, 50 ng/mm, 60 ng/mm, 70 ng/mm, 80 ng/mm, 90 ng/mm, 100ng/mm, 200 ng/mm, 300 ng/mm, 400 ng/mm, 500 ng/mm, 600 ng/mm, 700 ng/mm, 800 ng/mm, 900 ng/mm, 1 µg/mm, 2 µg/mm, 3 µg/mm, 4 µg/mm, 5 µg/mm, 6 µg/mm, 7 µg/mm, 8 µg/mm, 9 µg/mm, or 10 µg/mm diseased segment of the blood vessel. The statin can also be administered at a dosage ranging between any of the values described above.

In some embodiments, acute administration of the statin involves delivery of at least 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% of the statin dosage within 1, 2, 3, 4, 5, 6, 7, 8, 9, or 10 minutes.

Acute statin delivery can be achieved using known percutaneous transluminal devices, such as balloon catheters. In some cases, the statin can be delivered using a device that delivers the statin into the lumen of the blood vessel. The statin can subsequently contact and crosses the endothelium of the blood vessel. The statin can also be delivered using a device that perforates the endothelium of the blood vessel, and delivers the statin into the endothelial cells and/or smooth muscle cells.

For example, methods can first involve advancing a catheter through a blood vessel to the diseased segment of a blood vessel. In some cases, this involves first advancing a guide wire to the target site, and then advancing the catheter over the wire. Once positioned, a balloon can be inflated so that it delivers the statin to the wall of the diseased segment.

In some cases, the formulation can be administered locally to a segment of a blood vessel using a porous balloon catheter (sometimes referred to as a 'weeping balloon catheter'). In these embodiments, the method can involve percutaneously administering to the subject a porous balloon catheter which comprises (i) a proximal region; (ii) a distal region comprising a distal tip; (iii) a porous balloon for drug delivery positioned in proximity to the distal tip; transluminally advancing the catheter until the porous balloon reaches a diseased segment of a blood vessel in the subject; and injecting a pharmaceutical formulation comprising a statin into the diseased segment of a blood vessel through the porous balloon.

For example, the porous balloon can be a multiple-balloon fluid delivery catheter having a catheter shaft having a perforated, expandable outer balloon disposed around at least a portion of an inflatable inner balloon. The balloons in the respective inflated and expanded configurations define an annular lumen. The annular lumen can be in communication with a fluid delivery lumen extending along the catheter shaft and the holes of the outer balloon. Fluid passed through the fluid delivery lumen in the catheter shaft and the annular lumen may be released through the holes of the outer balloon.

A variety of porous balloon catheters are known in the art. *See,* for example, U.S. Patent No. 5,213,576 to Abiuso et al., U.S. Patent No. 8,034,022 to Boatman et al., U.S. Patent No. 8,591,461 to Boatman et al., U.S. Patent No. 8,182,446 to Schaeffer et al., U.S. Patent No. 8,066,690 to Vigil et al., and U.S. Patent No. 8,317,747 to Kusleika, U.S. Patent No. 8,211,055 to Christiansen, and U.S. Patent No. 8,715,230 to Baumbach et al. Suitable porous balloon catheters also include commercially available porous balloon catheters, such as the CLEARWAY^{™} OTW liquid drug delivery balloon commercially available from Atrium Medical Corporation (Hudson, NH),

In some cases, the formulation can be administered locally to a segment of a blood vessel using a drug-coated balloon catheter. In some embodiments, the statin is coated on the surface of the balloon but is not bioavailable while the balloon is deflated. However, upon inflation of the balloon (e.g., causing it to unfold), the statin can be exposed to the aqueous environment, allowing it to release from the surface of the balloon into the lumen. In some cases the statin is delivered to the diseased segment when the balloon is inflated to the diameter of the lumen and the surface of the balloon contacts the epithelium. Therefore, in these embodiments, the method can involve percutaneously administering to the subject a balloon catheter which comprises (i) a proximal region; (ii) a distal region comprising a distal tip; (iii) a balloon coated on its outer surface with a pharmaceutical formulation comprising a statin positioned in proximity to the distal tip; transluminally advancing the catheter until the drug-coated balloon reaches a diseased segment of a blood vessel in the subject; and inflating the balloon.

A variety of drug-coated balloon catheters configured to locally administer an active agent to a segment of a blood vessel are known in the art. *See,* for example, U.S. Patent No. 8,480,620 to Stankus et al., U.S. Patent No. 8,617,104 to Yribarren et al., U.S. Patent No. 8,617,114 to Von Oepen et al., U.S. Patent No. 8,187,223 to Spargias, U.S. Patent No. 8,740,843 to Eaton et al., U.S. Patent Application Publication No. 2014/0046254 to Stankus et al., U.S. Patent Application Publication No. 2012/0078227 to Kangas, U.S. Patent Application Publication No. 2010/0286608 to Tittelbach et al., U.S. Patent Application Publication No. 2014/0171912 to Gharib et al., U.S. Patent Application Publication No. 2012/0143132 to Orlowski et al., and U.S. Patent Application Publication No. 2010/0292641 to Wijay et al.

Suitable catheters also include commercially available drug-coated balloon catheters, such as the ADVANCE^{®} 18 PTX drug-eluting balloon dilation catheter commercially available from Cook Medical (a paclitaxel-coated balloon), modified to include a statin formulation in place of the drug coating they are sold with.

In some cases, the formulation can be administered locally to a segment of a blood vessel using a transluminal catheter having at least one balloon injection feature. In these embodiments, the method can involve percutaneously administering to the subject a balloon catheter which comprises (i) a proximal region; (ii) a distal region comprising a distal tip; (iii) a balloon having an outer surface positioned in proximity to the distal tip; and (iv) one or more injection features affixed to the outer surface of the balloon; transluminally advancing the catheter until the balloon reaches a diseased segment of a blood vessel in the subject; inflating the balloon until the balloon surface contacts the diseased segment of a blood vessel; and injecting a pharmaceutical formulation comprising a statin through the injection features into the diseased segment of the blood vessel.

A variety of balloon catheters for injecting a formulation on or into the wall of a diseased segment of a blood vessel are known in the art. See, for example, U.S. Patent No. 5,746,716 to Vigil et al., U.S. Patent No. 5,364,356 to Hofling, U.S. Patent No. 8,357,118 to Orr, and U.S. Patent No. 5,833,659 to Kranys.

Suitable catheters also include commercially available catheters for locally administering active agents to a segment of a blood vessel, such as the CRICKET^{™} and BULLFROG^{®} micro-infusion catheters commercially available from Metcator Systems.

In the above and other embodiments, occlusion balloons can be used to block blood flow such that statin released from the catheter within the lumen of the blood vessel remains at the site of the diseased segment until it has been effectively delivered into the vessel wall. For example, in some cases, once the catheter is positioned, at least one balloon is inflated upstream, downstream, or both, from the diseased segment. The statin can then be released from the catheter into the lumen of the blood vessel containing the diseased segment. See, for example, U.S. Patent No. 8,262,611 to Teeslink et al.

Suitable catheters also include commercially available occlusion balloons catheters for locally administering active agents to a segment of a blood vessel, such as the GENIE^{®} drug delivery catheter commercially available from Acrostak, and the OCCLUSION PERFUSION CATHETER^{®} commercially available from Advanced Catheter Therapies.

In some cases, the method involves delivering the statin to a site implanted with a stent, e.g., to treat or prevent restenosis. For example, a catheter can be advanced into the lumen of the stent, and a balloon inflated to deliver the statin to vessel wall contacting the stent. This method can be used with any stent that can result in vascular disease, including, but not limited to bare metal stents, nitinol stents, and drug-eluting stents (e.g., sirolimus-eluting stents or paclitaxel-eluting stents). Where there is a bifurcation lesion, two-stent strategies exist, including T-stenting, V-stenting, simultaneous kissing stents, the crush, and the culotte.

### Statins

The methods described herein relate to treating vascular disease in a subject by administering a statin. Methods of treatment are not part of the claims.

The kit of the present invention comprises a pharmaceutical composition comprising a statin encapsulated in lipid-based particles. The statin comprises cerivastatin, or a pharmaceutically acceptable salt, prodrug, clathrate, or solvate thereof that inhibits HMG-CoA (3-hydroxy-3-methylglutaryl-coenzyme A) reductase, an enzyme thought to facilitate the synthesis cholesterol. More particularly, statins inhibit the conversion of HMG-CoA reductase to mevalonate, a precursor of sterols, including cholesterol. Since the enzyme used to make cholesterol is blocked by the statin, then less cholesterol is made. Cholesterol and triglycerides circulate in the bloodstream as part of lipoprotein complexes. Hence, statins generally lower lipids in this way.

Additionally, statins can also treat the endothelial dysfunction associated with vascular disease. Endothelial dysfunction is a systemic pathological state of the endothelium (the inner lining of blood vessels), and can be broadly defined as an imbalance between vasodilating and vasoconstricting substances produced by (or acting on) the endothelium. In many cases, endothelial dysfunction is attributable to the reduced bioavailability of vascodilators, such as nitric oxide (NO).

NO is biosynthesized by nitric oxide synthases (NOS), which convert L-arginine to L-citrulline, releasing nitric oxide. Asymmetric dimethylarginine (ADMA) is an endogenous inhibitor of nitric oxide synthase (NOS), and elevated levels of ADMA have been linked to vascular diseases, including atherosclerosis and restenosis. Because ADMA inhibits the activity of nitric oxide synthases *in vivo,* increased ADMA levels decrease the amount of the vascodilator NO in a subject, resulting in endothelial dysfunction and associated vascular diseases.

ADMA levels *in vivo* are regulated by dimethylarginine dimethylaminohydrolase (DDAH), which metabolizes ADMA. The enzyme DDAH2 represents the predominant endothelial DDAH isoform. When administered to a subject, statins can induce DDAH2 activity in the vascular endothelial cells of a subject, lowering ADMA levels, and resulting in an increase in NO levels and a decrease in endothelial dysfunction (and associated vascular diseases). In particular, some statins can selectively induce DDAH2 as compared to DDAH1.

Other factors, including inflammatory pathways, are also known to contribute to the vascular remodeling associated with vascular disease processes. The chemokine receptor CCR2 is known to play a central role in the establishment and maintenance of chronic inflammatory processes. CCR2 and its ligands (such CC chemokine ligand 2 (CCL2), also known as macrophage chemoattractant protein-1 (MCP-1)) represent a critical signaling pathway responsible for the recruitment of peripheral blood monocytes to locations of immune-mediated inflammation. Once recruited, these monocytes become inflammatory macrophages which function to establish and maintain a chronic inflammatory state. Studies have demonstrated that CCR2 can be overexpressed in the vascular endothelial cells in diseased segments of blood vessels, resulting in a chronic inflammatory response in the blood vessel. This can further contribute to the vascular remodeling associated with vascular disease processes. When administered to a subject, statins can reduce CCR2 expression in the vascular endothelial cells of a subject, decreasing the resulting chronic inflammatory response in the blood vessel, thereby treating the vascular disease.

In particular, some statins can simultaneously induce DDAH2 activity in the vascular endothelial cells of a subject, and reduce CCR2 expression in the vascular endothelial cells of a subject.

Various statins are known in the art. Examples of statins include atorvastatin (e.g., Atorvastatin Calcium, marketed under the trademark LIPITOR^{®} by Pfizer, Inc.), pravastatin (e.g., Pravastatin Calcium, marketed under the trademark PRAVACHOL^{®} by Bristol-Myers Squibb), fluvastatin (e.g., Fluvastatin Sodium, marketed under the trademark LESCOL^{®} by Novartis), cerivastatin (e.g., Cerivastatin Sodium, previously marketed under the trademark BAYCOL^{®} by Bayer Corporation), lovastatin (e.g., Lovastatin, marketed under the trademark MEVACOR^{®} by Merck & Co., Inc.), simvastatin (e.g., Simvastatin, marketed under the trademark ZOCOR^{®} by Merck & Co., Inc.), rosuvastatin (e.g., Rosuvastatin Calcium, marketed under the trademark CRESTOR^{®} by AstraZeneca, Inc.), mevastatin, pitavastatin, pravastatin, nisvastatin, and itavastatin.

Methods of making statins or isolating statins are known in the art. Statins can be made synthetically, or isolated from a bacteria (e.g., *Aspergillus terreus).* Methods of making statins, their chemical formulas and their properties are described, for example, in U.S. Patent No. 3,983,140 (e.g., mevastatin); U.S. Patent No. 4,231,938 (e.g., lovastatin); U.S. Patent No. 4,346,227 (e.g., pravastatin); U.S. Patent No. 4,448,784 (e.g., simvastatin); U.S. Patent No. 4,450,171 (e.g., simvastatin); U.S. Patent No. 5,354,772 (e.g., fluvastatin); U.S. Patent No. 5,006,530 (e.g., cerivastatin); U.S. Patent No. 5,177,080 (e.g., cerivastatin); U.S. Patent No. 4,681,893 (e.g., atorvastatin); U.S. Patent No. 5,273,995 (e.g., atorvastatin); U.S. Patent No. 5,385,929 (e.g., atorvastatin); U.S. Patent No. 5,686,104 (e.g., atorvastatin); U.S. Patent No. 5,260,440 (e.g., rosuvastatin); and U.S. Patent No. 5,011,930 (e.g., pitavastatin).

In the embodiments of the present invention , the statin comprises cerivastatin, or a pharmaceutically acceptable prodrug, salt, solvate, or clathrate thereof. "Cerivastatin", as used herein, refers to (3R,5*S*,6*E*)-7-[4-(4-fluorophenyl)-5-(methoxymethyl)-2,6-bis(propan-2-yl)pyridin-3-yl]-3,5-dihydroxyhept-6-enoic acid, the structure of which is shown below.

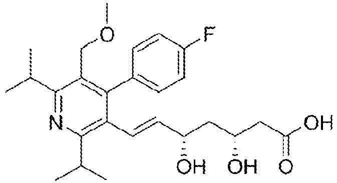

Analogs of cerivastatin include structurally related compounds which display similar pharmacological activity when administered to a patient in need thereof. Analogs include substituted 2,6-dialkyl-4-aryl-pyridines, such as those described in U.S. Patent Nos. 5,006,530, 5,169,857, 5,401,746, and 5,117,080 to Angerbauer, et al.

Examples of cerivastatin analogs include erythro-(E)-7-[2-(4-fluorophenyl)-5-hydroxymethyl-4-isopropyl-6-methyl-pyr id-3-yl]-3,5-dihydroxy-hept-6-enoate, erythro-(E)-7-[2-(4-fluorophenyl)-5-hydroxymethyl-4,6-diisopropyl-pyridin- 3-yl]-3,5-dihydroxy-hept-6-enoate, methyl erythro-(E)-7-[2,6-diisopropyl-4-(4-fluorophenyl)-5-methoxymethyl-pyrid-3-yl]-3,5-dihydroxy-hept-6enoate, methyl erythro-(E)-7-[5-tert.butyldimethylsiloxymethyl-4-(4-fluorophenyl)-2-isopr opyl-6methyl-pyrid-3-yl]-3,5-dihydroxy-hept-6-enoate, methyl erythro-(E)-7-[3-benzyloxymethyl-4-(4-fluorophenyl)-6-isopropyl-2-methyl-p yrid-5-yl]-3,5-dihydroxy-hept-6-enoate, methyl erythro-(E)-7-[5-tert.-butyldimethylsilyloxymethyl-2,6-dimethyl-4-(4-fluor ophenyl)pyrid-3-yl]-3,5-dihydroxy-hept-6-enoate, methyl erythro-(E)-7-[6-cyclopropyl-2-isopropyl-4-(4-fluorophenyl)-5-methoxymethy 1-pyrid-3-yl]-3,5-dihydroxy-hept-6-enoate, methyl erythro-(E)-7-[2,6-diisopropyl-4-(4-fluorophenyl)-5-phenoxymethyl-pyrid-3- yl]-3,5-dihydroxy-hept-6-enoate, methyl erythro-(E)-7-[2,6-diisopropyl-4-(4-fluorophenyl)-5-carboxymethyl-pyrid-3- yl]-3,5-dihydroxy-hept-6-enoate, methyl erythro-(E)-7-[2,6-diisopropyl-4-(4-fluorophenyl)-5-benzothio-methyl-pyrid -3-yl]-3,5-dihydroxy-hept-6-enoate, methyl erythro-(E)-7-[2,6-diisopropyl-5-ethoxymethyl-4-(4-fluorophenyl)-pyrid-3-yl]-3,5-dihydroxy-hept-6-enoate, methyl erythro-(E)-7-[2,6-diisopropyl-4-(4-fluorophenyl)-5-propyloxymethyl-pyrid- 3-yl]-3,5-dihydroxy-hept-6-enoate, methyl erythro-(E)-7-[2,6-diisopropyl-4-(4-fluorophenyl)-5-isopropoxymethyl-pyrid -3-yl]-3,5-dihydroxy-hept-6-enoate, methyl erythro-(E)-7-[2,6-diisopropyl-4-(4-fluorophenyl)-5-butyloxymethyl-pyrid-3 -yl]-3,5-dihydroxy-hept-6-enoate, methyl erythro-(E)-7-[2,6-diisopropyl-4-(4-fluorophenyl)-5-pentyloxymethyl-pyrid- 3-yl]-3,5-dihydroxy-hept-6-enoate, methyl erythro-(E)-7-[2,6-diisopropyl-4-(4-fluorophenyl)-5-hexyloxymethyl-pyrid-3 -yl]-3,5-dihydroxy-hept-6-enoate, and methyl erythro-(E)-7-[2,6-diisopropyl-3-(4-fluorobenzyloxymethyl)-4-(4-fluropheny l-pyrid-5-yl] -3,5-dihydroxy-hept-6-enoate.

The statin can also comprise a pharmaceutically acceptable prodrug of cerivastatin. Prodrugs of cerivastatin are compounds that, when metabolized *in vivo,* undergo conversion to compounds having the desired pharmacological activity. Prodrugs can be prepared by replacing appropriate functionalities present in cerivastatin or a cerivastatin analog with "pro-moieties" as described, for example, in H. Bundgaar, Design of Prodrugs (1985). Examples of prodrugs include ester, ether or amide derivatives of cerivastatin, and their pharmaceutically acceptable salts.

For further discussions of prodrugs, see, for example, T. Higuchi and V. Stella "Pro-drugs as Novel Delivery Systems," ACS Symposium Series 14 (1975) and E. B. Roche ed., Bioreversible Carriers in Drug Design (1987).

The statin can also comprise a pharmaceutically acceptable salt of cerivastatin.

In some cases, it may be desirable to administer a salt of cerivastatin due to one or more of the salt's advantageous physical properties, such as enhanced stability or a desirable solubility or dissolution profile.

Generally, pharmaceutically acceptable salts of cerivastatin can be prepared by reaction of the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are preferred. Lists of suitable salts are found, for example, in Remington's Pharmaceutical Sciences, 20th ed., Lippincott Williams & Wilkins, Baltimore, MD, 2000, p. 704.

Suitable pharmaceutically acceptable acid addition salts of the compounds of the present invention when possible include those derived from inorganic acids, such as hydrochloric, hydrobromic, hydrofluoric, boric, fluoroboric, phosphoric, metaphosphoric, nitric, carbonic, sulfonic, and sulfuric acids, and organic acids such as acetic, benzenesulfonic, benzoic, citric, ethanesulfonic, fumaric, gluconic, glycolic, isothionic, lactic, lactobionic, maleic, malic, methanesulfonic, trifluoromethanesulfonic, succinic, toluenesulfonic, tartaric, and trifluoroacetic acids.

Suitable organic acids generally include, for example, aliphatic, cycloaliphatic, aromatic, araliphatic, heterocyclic, carboxylic, and sulfonic classes of organic acids. Specific examples of suitable organic acids include acetate, trifluoroacetate, formate, propionate, succinate, glycolate, gluconate, digluconate, lactate, malate, tartaric acid, citrate, ascorbate, glucuronate, maleate, fumarate, pyruvate, aspartate, glutamate, benzoate, anthranilic acid, mesylate, stearate, salicylate, *p*-hydroxybenzoate, phenylacetate, mandelate, embonate (pamoate), methanesulfonate, ethanesulfonate, benzenesulfonate, pantothenate, toluenesulfonate, 2-hydroxyethanesulfonate, sufanilate, cyclohexylaminosulfonate, algenic acid, β-hydroxybutyric acid, galactarate, galacturonate, adipate, alginate, butyrate, camphorate, camphorsulfonate, cyclopentanepropionate, dodecylsulfate, glycoheptanoate, glycerophosphate, heptanoate, hexanoate, nicotinate, 2-naphthalesulfonate, oxalate, palmoate, pectinate, 3-phenylpropionate, picrate, pivalate, thiocyanate, tosylate, and undecanoate.

In some cases, the pharmaceutically acceptable salt of cerivastatin may include alkali metal salts, including but not limited to sodium or potassium salts; alkaline earth metal salts, e.g., calcium or magnesium salts; and salts formed with suitable organic ligands, e.g., quaternary ammonium salts. In another embodiment, base salts are formed from bases which form non-toxic salts, including aluminum, arginine, benzathine, choline, diethylamine, diolamine, glycine, lysine, meglumine, olamine, tromethamine and zinc salts.

Organic salts may be made from secondary, tertiary or quaternary amine salts, such as tromethamine, diethylamine, N,N'-dibenzylethylenediamine, chloroprocaine, choline, diethanolamine, ethylenediamine, meglumine (N-methylglucamine), and procaine. Basic nitrogen-containing groups may be quaternized with agents such as lower alkyl (C₁-C₆) halides (*e.g.,* methyl, ethyl, propyl, and butyl chlorides, bromides, and iodides), dialkyl sulfates (*e.g.,* dimethyl, diethyl, dibuytl, and diamyl sulfates), long chain halides (e.g., decyl, lauryl, myristyl, and stearyl chlorides, bromides, and iodides), arylalkyl halides (e.g., benzyl and phenethyl bromides), and others.

The statin can also comprise a pharmaceutically acceptable solvate of cerivastatin.

Solvates of cerivastatin include molecular complexes formed between cerivastatin and one or more pharmaceutically acceptable solvent molecules (e.g., ethanol). The term "hydrate" refers to solvates in which the pharmaceutically acceptable solvent is water. A currently accepted classification system for solvates of organic compounds is one that distinguishes between isolated site, channel, and metal-ion coordinated solvates and hydrates. See, for example, K. R. Morris (H. G. Brittain ed.) *Polymorphism in Pharmaceutical Solids,* Marcel Dekker Inc., New York, NY (1995). Isolated site solvates are solvates in which the solvent molecules are isolated from direct contact with each other by intervening molecules of the organic compound. In channel solvates, the solvent molecules lie in lattice channels where they are next to other solvent molecules. In metal-ion coordinated solvates, the solvent molecules are bonded to the metal ion.

When the solvent is tightly bound, the solvate will have a well-defined stoichiometry independent of ambient humidity. However, when the solvent is weakly bound, as in channel solvates and in hygroscopic compounds, the solvent content will depend on humidity and drying conditions. In such cases, the solvate will generally incorporate solvent molecules in a non-stoichiometric ratio.

The statin can also comprise a pharmaceutically acceptable clathrate of cerivastatin.

Clathrates are drug-host inclusion complexes formed when a drug is associated with or in a host molecule or molecules in stoichiometric ratio. For example, cerivastatin can form inclusion complexes with cyclodextrins or other host molecules.

The statin can also comprise pharmaceutically acceptable co-crystals of cerivastatin.

Co-crystals are crystalline complexes of two or more molecular constituents, one of which is cerivastatin.

The molecular constituents may be two or more neutral molecules, two or more salts, or a complex containing one or more neutral molecules and one or more salts. Co-crystals may be prepared by melt crystallization, recrystallization from solvents, or by physically mixing the components together, for example, by grinding. See, e.g., O. Almarsson and M. J. Zaworotko, Chem. Commun., 17:1889-1896 (2004). Examples of multi-component complexes are well known in the art. See, for example, J. K. Haleblian, J. Pharm. Sci. 64(8):1269-88 (1975).

Cerivastatin, as well as pharmaceutically acceptable prodrugs or salts thereof, may contain one or more chiral centers, and thus exist as one or more stereoisomers. Such stereoisomers can be prepared and/or isolated as a single enantiomer, a mixture of diastereomers, or a racemic mixture. Choice of the appropriate chiral column, eluent, and conditions necessary to effect separation of the pair of enantiomers is well known to one of ordinary skill in the art using standard techniques (see e.g. Jacques, J. et al., "Enantiomers, Racemates, and Resolutions", John Wiley and Sons, Inc. 1981).

The statin can also comprise a combination than one of the following: cerivastatin, a prodrug of cerivastatin, a salt of cerivastatin, a salvate of cerivastatin, a clathrate of cerivastatin.

The kit of claim 1 may also be combined with other therapeutic agents suitable for treating vascular disease. For example, in some embodiments, cerivastatin, or a pharmaceutically acceptable salt, prodrug, clathrate, or solvate thereof are administered in combination with cellular therapies, such as pluripotent stem cells or endothelial progenitor cells.

### Pharmaceutical Formulations

Statins can be appropriately formulated for delivery using methods known in the art. The nature of the pharmaceutical formulations can vary depending upon a number of factors, including the statin(s) being administered, the vascular disease being treated, considerations regarding the subject being treated, and the method by which the statin is locally administered to the diseased segment of a blood vessel in the subject (e.g., whether the statin is locally administered to the diseased segment of the blood vessel via a porous balloon catheter, a drug-coated balloon catheter, or via a catheter for injecting a formulation on or into a wall of the diseased segment of the blood vessel). For example, statins can be formulated as injectable formulations for administration using a porous balloon catheter, or as solid formulations, gels, or liquids to be administered using a drug-coated balloon catheter.

Pharmaceutical formulations can include a therapeutically effective amount of one or more statins in combination with one or more pharmaceutically acceptable excipients. Representative excipients include solvents, diluents, pH modifying agents, preservatives, antioxidants, suspending agents, wetting agents, viscosity modifiers, tonicity agents, stabilizing agents, and combinations thereof. Suitable pharmaceutically acceptable excipients are preferably selected from materials that are generally recognized as safe (GRAS) by the FDA, and can be administered to an individual without causing undesirable biological side effects or unwanted interactions. If desired, the formulations can further include (or be co-administered with) other standard-of-care drugs for the treatment of vascular diseases.

Suitable pharmaceutical formulations can include standard parenteral formulations, including injectable formulations, for example, solutions or suspensions; solid forms suitable for using to prepare solutions or suspensions upon the addition of a reconstitution medium prior to injection; emulsions, such as water-in-oil (w/o) emulsions, oil-in-water (o/w) emulsions, and microemulsions thereof, liposomes, and emulsomes.

The carrier can be a solvent or dispersion medium containing, for example, water, ethanol, one or more polyols (e.g., glycerol, propylene glycol, and liquid polyethylene glycol), oils, such as vegetable oils (e.g., peanut oil, corn oil, sesame oil, etc.), and combinations thereof.

The proper fluidity can be maintained, for example, by the use of a coating, such as lecithin, by the maintenance of the required particle size in the case of dispersion and/or by the use of surfactants. In many cases, it will be preferable to include isotonic agents, for example, sugars or sodium chloride.

Solutions and dispersions of the active compounds as the free acid or base or pharmacologically acceptable salts thereof can be prepared in water or another solvent or dispersing medium suitably mixed with one or more pharmaceutically acceptable excipients including, but not limited to, surfactants, dispersants, emulsifiers, pH modifying agents, and combination thereof.

Suitable surfactants may be anionic, cationic, amphoteric or nonionic surface active agents. Suitable anionic surfactants include, but are not limited to, those containing carboxylate, sulfonate and sulfate ions. Examples of anionic surfactants include sodium, potassium, ammonium of long chain alkyl sulfonates and alkyl aryl sulfonates such as sodium dodecylbenzene sulfonate; dialkyl sodium sulfosuccinates, such as sodium dodecylbenzene sulfonate; dialkyl sodium sulfosuccinates, such as sodium bis-(2-ethylthioxyl)-sulfosuccinate; and alkyl sulfates such as sodium lauryl sulfate. Cationic surfactants include, but are not limited to, quaternary ammonium compounds such as benzalkonium chloride, benzethonium chloride, cetrimonium bromide, stearyl dimethylbenzyl ammonium chloride, polyoxyethylene and coconut amine. Examples of nonionic surfactants include ethylene glycol monostearate, propylene glycol myristate, glyceryl monostearate, glyceryl stearate, polyglyceryl-4-oleate, sorbitan acylate, sucrose acylate, PEG-150 laurate, PEG-400 monolaurate, polyoxyethylene monolaurate, polysorbates, polyoxyethylene octylphenylether, PEG-1000 cetyl ether, polyoxyethylene tridecyl ether, polypropylene glycol butyl ether, Poloxamer^{®} 401, stearoyl monoisopropanolamide, and polyoxyethylene hydrogenated tallow amide. Examples of amphoteric surfactants include sodium N-dodecyl-β-alanine, sodium N-lauryl-β-iminodipropionate, myristoamphoacetate, lauryl betaine and lauryl sulfobetaine.

The formulation can contain a preservative to prevent the growth of microorganisms. Suitable preservatives include, but are not limited to, parabens, chlorobutanol, phenol, sorbic acid, and thimerosal. The formulation may also contain an antioxidant to prevent degradation of the active agent(s).

The formulation is typically buffered to a pH of 3-8 for administration upon reconstitution. Suitable buffers include, but are not limited to, phosphate buffers, acetate buffers, and citrate buffers.

If desired, water soluble polymers can be included in these formulations. Suitable watersoluble polymers include, but are not limited to, polyvinylpyrrolidone, dextran, carboxymethylcellulose, and polyethylene glycol.

Sterile injectable solutions can be prepared by incorporating the active compounds in the required amount in the appropriate solvent or dispersion medium with one or more of the excipients listed above, as required, followed by filtered sterilization. Generally, dispersions are prepared by incorporating the various sterilized active ingredients into a sterile vehicle which contains the basic dispersion medium and the required other ingredients from those listed above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. The powders can be prepared in such a manner that the particles are porous in nature, which can increase dissolution of the particles. Methods for making porous particles are well known in the art.

Parenteral formulations can be formulated for controlled release including immediate release, delayed release, extended release, pulsatile release, and combinations thereof. For example, statins, and optionally one or more additional active agents, can be incorporated into microparticles, nanoparticles, or combinations thereof that provide controlled release. In embodiments wherein the formulations contains two or more drugs, the drugs can be formulated for the same type of controlled release (e.g., delayed, extended, immediate, or pulsatile) or the drugs can be independently formulated for different types of release (e.g., immediate and delayed, immediate and extended, delayed and extended, delayed and pulsatile, etc.).

As discussed in more detail below, in the embodiments according to the invention, the formulation comprises a statin encapsulated in a lipid-based particle, such as a liposome or a lipid micelle, wherein the statin comprises cerivastatin, or a pharmaceutically acceptable salt, prodrug, clathrate, or solvate thereof. In certain embodiments, the lipid-based particle can have an average particle size of from 1 nm to 200 nm (e.g., from 5 nm to 50 nm, from 5 nm to 100 nm, from 50 nm to 100 nm, from 100 nm to 150 nm, from 100 nm to 200 nm, or from 150 nm to 200 nm), as determined by dynamic light scattering.

### Liposomal Formulations

In certain embodiments, the pharmaceutical formulation can be a liposomal formulation. Liposomal formulations of statins can include liposomes formed from a vesicle-forming lipid, and a statin entrapped in the liposomes. A statin entrapped in a liposome can be sequestered in the central aqueous compartment of the liposome, in the aqueous space between liposome lipid bilayers, or within a bilayer of the liposome itself.

The liposomes in the formulation can be composed primarily of vesicle-forming lipids. Such a vesicle-forming lipid is one that can form spontaneously into bilayer vesicles in water, as exemplified by the phospholipids, with its hydrophobic moiety in contact with the interior, hydrophobic region of the bilayer membrane, and its head group moiety oriented toward the exterior, polar surface of the membrane. Lipids capable of stable incorporation into lipid bilayers, such as cholesterol and its various analogs, can also be used in the liposomes. The vesicle-forming lipids are preferably lipids having two hydrocarbon chains, typically acyl chains, and a head group, either polar or nonpolar. There are a variety of synthetic vesicle-forming lipids and naturally-occurring vesicle-forming lipids, including the phospholipids, such as phosphatidylcholine, phosphatidylethanolamine, phosphatidic acid, phosphatidylinositol, and sphingomyelin, where the two hydrocarbon chains are typically between about 14-22 carbon atoms in length, and have varying degrees of unsaturation. The above-described lipids and phospholipids whose acyl chains have varying degrees of saturation can be obtained commercially or prepared according to published methods. Other suitable lipids include glycolipids, cerebrosides and sterols, such as cholesterol.

The vesicle-forming lipid can be selected to achieve a specified degree of fluidity or rigidity, to control the stability of the liposome in serum, and/or to control the rate of release of the entrapped agent in the liposome. Liposomes having a more rigid lipid bilayer, or a liquid crystalline bilayer, are achieved by incorporation of a relatively rigid lipid, *e.g.,* a lipid having a relatively high phase transition temperature, *e.g.,* up to 60° C. Rigid, *i.e.,* saturated, lipids contribute to greater membrane rigidity in the lipid bilayer. Other lipid components, such as cholesterol, are also known to contribute to membrane rigidity in lipid bilayer structures. On the other hand, lipid fluidity is achieved by incorporation of a relatively fluid lipid, typically one having a lipid phase with a relatively low liquid to liquid-crystalline phase transition temperature, e.g., at or below room temperature.

The liposomes can optionally include a vesicle-forming lipid covalently linked to a hydrophilic polymer. As described, for example in U.S. Patent No. 5,013,556, including such a polymer-derivatized lipid in the liposome composition forms a surface coating of hydrophilic polymer chains around the liposome. The surface coating of hydrophilic polymer chains is effective to increase the in vivo blood circulation lifetime of the liposomes when compared to liposomes lacking such a coating. Polymer-derivatized lipids comprised of methoxy(polyethylene glycol) (mPEG) and a phosphatidylethanolamine (e.g., dimyristoyl phosphatidylethanolamine, dipalmitoyl phosphatidylethanolamine, distearoyl phosphatidylethanolamine (DSPE), or dioleoyl phosphatidylethanolamine) can be obtained from Avanti Polar Lipids, Inc. (Alabaster, Ala.) at various mPEG molecular weights (350, 550, 750, 1,000, 2,000, 3,000, and 5,000 Daltons). Lipopolymers of mPEG-ceramide can also be purchased from Avanti Polar Lipids, Inc. Preparation of lipid-polymer conjugates is also described in the literature, see U.S. Pat. Nos. 5,631,018, 6,586,001, and 5,013,556; Zalipsky, S. et al., Bioconjugate Chem. 8:111 (1997); Zalipsky, S. et al., Meth. Enzymol. 387:50 (2004). These lipopolymers can be prepared as well-defined, homogeneous materials of high purity, with minimal molecular weight dispersity (Zalipsky, S. et al., Bioconjugate Chem. 8:111 (1997); Wong, J. et al., Science 275:820 (1997)). The lipopolymer can also be a "neutral" lipopolymer, such as a polymer-distearoyl conjugate, as described in U.S. Pat. No. 6,586,001.

When a lipid-polymer conjugate is included in the liposomes, typically between 1-20 mole percent of the lipid-polymer conjugate is incorporated into the total lipid mixture (see, *e.g.,* U.S. Patent No. 5,013,556).

If desired for a particular application, the liposomes can include a ligand, such as a targeting ligand, conjugated to the liposome. For example, the liposomes can optionally include a lipopolymer modified to include a ligand, forming a lipid-polymer-ligand conjugate, also referred to herein as a 'lipopolymer-ligand conjugate'. The ligand can be a therapeutic molecule, such as a drug or a biological molecule having activity *in vivo,* a diagnostic molecule, such as a contrast agent or a biological molecule, or a targeting molecule having binding affinity for a binding partner, preferably a binding partner on the surface of a cell. For example, the ligand can have binding affinity for the surface of a cell, so as to facilitate entry of the liposome into the cytoplasm of a cell via internalization. A ligand present in liposomes that include such a lipopolymer-ligand is oriented outwardly from the liposome surface, and therefore available for interaction with its cognate receptor.

Methods for attaching ligands to lipopolymers are known, where the polymer can be functionalized for subsequent reaction with a selected ligand. (U.S. Pat. No. 6,180,134; Zalipsky, S. et al.,FEBS Lett. 353:71 (1994); Zalipsky, S. et al.,Bioconjugate Chem. 4:296 (1993); Zalipsky, S. et al., J. Control. Rel. 39:153 (1996); Zalipsky, S. et al., Bioconjugate Chem. 8(2):111 (1997); Zalipsky, S. et al.,Meth. Enzymol. 387:50 (2004)). Functionalized polymer-lipid conjugates can also be obtained commercially, such as end-functionalized PEG-lipid conjugates (Avanti Polar Lipids, Inc.). The linkage between the ligand and the polymer can be a stable covalent linkage or a releasable linkage that is cleaved in response to a stimulus, such as a change in pH or presence of a reducing agent.

Liposomal formulations including statins can be formed using any suitable method for preparing and/or loading liposomes. For example, a compound described herein and one or more vesicle-forming lipids can be dissolved in a suitable solvent, and the solvent can be evaporated to form a lipid film. The lipid film can be hydrated with an aqueous solution (e.g., having a pH of from 7-9) to form liposomes comprising the entrapped compound.

After liposome formation, the liposomes can be sized to obtain a population of liposomes having a substantially homogeneous size range, for example from 0.01 to 0.5 microns (e.g., from 0.03-0.40 microns). Liposomes can be sized by any suitable method, such as by extrusion through a series of membranes having a selected uniform pore size (e.g., polycarbonate membranes having a selected uniform pore size in the range of 0.03 to 0.2 micron). The pore size of the membrane corresponds roughly to the largest sizes of liposomes produced by extrusion through that membrane, particularly where the preparation is extruded two or more times through the same membrane. Homogenization methods can also be used to prepare liposomes having sizes of 100 nm or less (Martin, F. J., in Specialized Drug Delivery Systems-Manufacturing and Production Technology, P. Tyle, Ed., Marcel Dekker, New York, pp. 267-316 (1990)). In certain embodiments, the formulation can comprise liposomes having an average particle size of from 5 nm to 500 nm, as measured by dynamic light scattering. In certain embodiments, the formulation can comprise liposomes having an average particle size of from 5 nm to 250 nm, as measured by dynamic light scattering. In certain embodiments, the formulation can comprise liposomes having an average particle size of from 5 nm to 100 nm, as measured by dynamic light scattering. In certain embodiments, the formulation can comprise liposomes having an average particle size of from 5 nm to 50 nm, as measured by dynamic light scattering.

After sizing, unencapsulated statin can be removed by a suitable technique, such as dialysis, centrifugation, size exclusion chromatography or ion exchange to achieve a suspension of liposomes having a high concentration of entrapped statin in the liposomes and little to no statin in solution outside of the liposomes. Also after liposome formation, the external phase of the liposomes can be adjusted, if desired, by titration, dialysis or the like, to an appropriate pH.

Once formed, the liposomal suspension can be lyophilized using methods known in the art. The resulting composition can be in the form of a lyophilized powder. The term "lyophilized powder" refers to any solid material obtained by lyophilization of an aqueous mixture. In some examples, a lyoprotectant, such as sucrose or trehalose, can be added to the liposomal formulation prior lyophilization.

Lyophilized formulations can be reconstituted prior to administration by adding an aqueous solvent. The reconstitution solvent can be suitable for pharmaceutical administration (*e.g.,* for parenteral administration to a subject). Examples of suitable reconstitution solvents include, without limitation, water, saline, and phosphate buffered saline (PBS).

### Lipid Emulsions

In certain embodiments, the pharmaceutical formulation can be a lipid emulsion. Statins can be combined suspended or dissolved using a lipid emulsion. Lipid emulsions are known in the art. *See,* for example, U.S. Patent No. 6361792 to Long, et. al*.*; U.S. Patent No. 7,550,155 to Zhang, et al.*,* and U.S. Patent Application Publication No. US 2006/0067952. Lipid emulsions formulations typically include one or more statins, an oil component, an emulsifier, and water.

The oil component can be a monoglyceride, a diglyceride, a triglyceride, or combinations thereof. In some cases, the oil component includes an ester formed between one or more fatty acids and an alcohol other than glycerol. The oil component can be, for example, a vegetable oil such as almond oil, borage oil, black currant seed oil, corn oil, safflower oil, soybean oil, sesame oil, cottonseed oil, peanut oil, olive oil, rapeseed oil, coconut oil, palm oil, canola oil, or combinations thereof. Vegetable oils are typically long-chain triglycerides formed from C₁₄-C₂₂ fatty acids. The oil component can also include medium chain triglycerides formed from C8-C12 fatty acids, such as Miglyol 812, Crodamol^{®} GTCC-PN, or Neobees M-5 oil.

The emulsifier serves to stabilize the lipid emulsion by preventing separation of the emulsion into individual oil and aqueous phases. Suitable emulsifiers include, but are not limited to, propylene glycol mono- and di-fatty acid esters, polyoxyethylene sorbitan fatty acid esters, polyoxyethylene fatty acid esters, polyoxyethylene-polyoxypropylene co-polymers and block co-polymers, salts of fatty alcohol sulphates, sorbitan fatty acid esters, esters of polyethylene-glycol glycerol ethers, oil and wax based emulsifiers, glycerol monostearate, glycerine sorbitan fatty acid esters and phospholipids. In some cases the emulsifier is a phospholipid.

In some cases, the emulsifier is a vitamin E derivative. Suitable vitamin E derivatives include, but are not limited to, α-tocopheryl oxalate, α-tocopheryl malonate, α-tocopheryl succinate, α-tocopheryl glutarate, α-tocopheryl adipate, α-tocopheryl pimelate, α-tocopheryl suberate, α-tocopheryl azelate, and D-α-tocopheryl polyethylene glycol 1000 succinate (vitamin E TPGS).

Exemplary phospholipids include, but are not limited to, phosphatidyl chlorine, lecithin (a mixture of choline ester of phosphorylated diacylglyceride), phosphatidylethanolamine, phosphatidylglycerol, phosphatidic acid with about 4 to about 22 carbon atoms, and more generally from about 10 to about 18 carbon atoms and varying degrees of saturation. Preferably, the phospholipid is of natural origin. Naturally occurring phospholipids include soy lecithin, egg lecithin, hydrogenated soy lecithin, hydrogenated egg lecithin, sphingosine, gangliosides, and phytosphingosine, and combinations thereof.

Suitable lipid emulsions generally contain between about 1% and 40% w/v oil component and between about 0.1% and 7.5% w/v emulsifier. Suitable commercially available lipid emulsions include lipid emulsions containing soybean oil, such as Intralipid^{®} 10%, Intralipid^{®} 20%, and Intralipid^{®} 30%, and as well as lipid emulsions containing a mixture of soybean and safflower oils, such as Liposyn^{®} II 10% and Liposyn^{®} II 20%.

Lipid emulsions can optionally contain one or more additional components. For example, lipid formulations can contain one or more non-aqueous miscible co-solvents, such as an alcohol or glycol. In some preferred formulations, glycerol and/or propylene glycol is present as a co-solvent.

Many lipid emulsions are capable of supporting bacterial growth. Accordingly, in some cases, one or more components may be added to the lipid emulsion formulation to prevent or retard bacterial growth, for example disodium edatate, citric acid, metabisulfate, benzyl alcohol, one or more parabens, chlorobutanol, phenol, sorbic acid, or thimerosal.

Additionally, lipid emulsions can contain one or more agents used to modify or stabilize the pH of the solution, including phosphate buffers, acetate buffers, and citrate buffers.

The pharmaceutical formulation can also be a lipid nanoemulsion. Lipid nanoemulsions are known in the art. *See,* for example, U.S. Patent Application Publication No. US 2007/0207173 to Chen, et al*,* and U.S. Patent Application Publication No. US 2001/0045050 to Elbayoumi, et al.

Lipid nanoemulsions can be prepared by microemulsification of any of the lipid emulsions described above using for example, a high pressure homogenizer, or via a phase inversion temperature method (PIT). In example lipid nanoemulsions containing statins, vitamin E succinate and/or Vitamin E TPGS are included as emulsifiers.

Therefore, in certain embodiments, the statin can be provided as a lipid micellular formulation comprising a lipid micelle, and a statin entrapped in the lipid micelle. The term "micelle," as used herein, refers to a core-shell structure composed of a monolayer of amphiphilic molecules, wherein in aqueous media, the shell is composed of the "hydrophilic head" regions of the amphiphilic molecules, and the core is composed of the hydrophobic tail regions of the amphiphilic molecules. This type of micelle is known as a normal phase micelle (also called oil-in-water micelle). Inverse micelles, also called water-in-oil micelles, have the hydrophilic heads at the center, with the hydrophobic tails extending out. In some embodiments, the lipid micelles can be oil-in-water micelles.. Micelles may be spherical or tubular or wormlike, and form spontaneously at or above the critical micelle concentration (CMC). In general, micelles are in equilibrium with the monomers under a given set of physical conditions such as temperature, ionic environment, concentration, etc.

Lipid micelles can be formed from any suitable micelle-forming compounds, including amphipathic lipids (e.g., vesicle-forming lipids as described herein or known in the art), with one or more additional components (e.g., lipoproteins, detergents, non-lipid polymers, etc.) incorporated into the micellar structure. In some embodiments, the lipid micelles can have an average particle size of less than 50 nm (e.g., from 5 nm to 50 nm), as determined by dynamic light scattering.

The lipid nanoemulsion (e.g., the lipid micelles) can further be lyophilized if desired. *See,* for example, U.S. Patent Publication No. US 2011/0015266. Nanoemulsions typically include an oil phase which has at least one fatty acid oil. Preferable polyunsaturated fatty acids include eicosapentaenoic acid, salts of eicosapentaenoic acid, docosahexaenoic acid, salts of docosahexaenoic acid, triglycerides of eicosapentaenoic acid, tryglycerides of docosahexaenoic acid, ethyl esters of eicosapentaenoic acid, or ethyl esters of docosahexaenoic acid. The nanoemulsion also includes at least one or more surfactants. Surfactants include any molecule having both a polar head group, which energetically prefers solvation by water, and a hydrophobic tail that is not well solvated by water. The ratio of the oil phase to the emulsifier component is important for the toxicity of the nanoemulsion prepared from the pre-concentrate.

The surfactant can be provided in either the aqueous or the oil phase. Surfactants suitable for use include a variety of anionic and nonionic surfactants, as well as other emulsifying compounds that are capable of promoting the formation of oil-in-water emulsions; so long as they are on the GRAS list and are approved for human consumption such as lecithin, solutol HS-15 (polyoxyethylene esters of 12-hydroxystearic acid), polysorbate 80 or Cremophore EL (polyethoxylated castor oil). In general, emulsifying compounds are relatively hydrophilic, and blends of emulsifying compounds can be used to achieve the necessary qualities. In some parenteral formulations, nonionic surfactants have advantages over ionic emulsifiers in that they are substantially more compatible with a broad pH range and often form more stable emulsions than do ionic (e.g., soap-type) emulsifiers. Non-ionic surfactants which are particularly preferred include polyethoxylated hydrogenated castor oil containing 35 mol of ethylene oxide (hereafter referred to as "with 35 EO"), polyethoxylated hydrogenated castor oil containing 7 mol of ethylene oxide (or with 7 EO), polyethoxylated olive oil with 7 EO, sorbitan monooleates with 4 EO, 5 EO or 20 EO, (C₁₂-C₁₄-alkyl)glycosides or (C₈-C₁₄alkyl)glycosides, glycerol monostearate with 30 EO, decaglyceryl monooleate, polyalkoxylated oleyl alcohol with 2 or 10 EO, polyethoxylated lauryl alcohol with 7 EO, methylglucoside dioleate, and mixtures thereof. Polyethoxylated castor oil-based emusifiers, such as cremophore EL or the less sensitizing solutol HS-15, are most preferred. Long chain ethoylated surfactants such as Cremophore EL 35, polyethoylated castor oil, a BASF product, TPGS 1000, polyethyleneglycol 1000 ester of alpha-tocopheryl succinate, TWEEN^{®} 80, or polyoxyethylene 20 sorbitan monooleate may also be employed as the surfactant. Vitamin E TPGS or PEGylated vitamin E in which polyethylene glycol subunits are attached by a succinic acid diester at the ring hydroxyl of the vitamin E molecule may also be used. TPGS stands for Dα-tocopherol polyethyleneglycol 1000 succinate (MW=530). TPGS is a non-ionic surfactant having an HLB value between 16 and 18.

HLB as used herein refers to the Hydrophile-Lipophile Balance Index Number and is an index for correlating the chemical structure of surfactant molecules with their surface activity. The HLB Index Number may be calculated by a variety of empirical formulas as described by Meyers, (Meyers, Surfactant Science and Technology, VCH Publishers Inc., New York, pp. 231-245 [1992]). The HLB Index Number of a surfactant is the HLB Index Number assigned to that surfactant in McCutcheon's Volume 1: Emulsifiers and Detergents North American Edition, 1996. The HLB Index Number ranges from 0 to about 20 or more for commercial surfactants. Hydrophilic surfactants with high solubility in water and solubilizing properties are at the high end of the scale, while surfactants with low solubility in water that are good solubilizers of water in oils are at the low end of the scale.

### Balloon Coatings

In some embodiments, the pharmaceutical composition comprising a statin encapsulated in lipid-based particles can be a coating formed on a surface of a drug-coated balloon catheter. The statin coating can be formed by spraying, dipping, pouring, pumping, brushing, wiping, vacuum deposition, vapor deposition, plasma deposition, electrostatic deposition, ultrasonic deposition, epitaxial growth, electrochemical deposition or any other method known to those skilled in the art.

The statin coating can comprise optionally one or more excipients and/or additives as described above. For example, the coating can include a biocompatible polymer. Suitable polymers can include both biostable and biodegradable polymers, such as microcrystalline cellulose, hydroxypropyl cellulose, hydroxypropyl methylcellulose, polyalkylene oxides such as polyethylene oxide (PEG), polyanhydrides, poly(ester anhydrides), polyhydroxy acids such as polylactide (PLA), polyglycolide (PGA), poly(lactide-co-glycolide) (PLGA), poly-3-hydroxybutyrate (PHB) and copolymers thereof, poly-4-hydroxybutyrate (P4HB) and copolymers thereof, polycaprolactone and copolymers thereof, and combinations thereof.

The coating can comprise rate-controlling excipients, including hydrophobic materials, including acceptable fats and fatty substances (e.g., fatty alcohols, such as lauryl, myristyl stearyl, cetyl or cetostearyl alcohol, fatty acids and derivatives, including, but not limited to, fatty acid esters, fatty acid glycerides (mono-, di- and tri-glycerides), and hydrogenated fats), waxes and wax-like substances (e.g., natural or synthetic waxes, hydrocarbons, and normal waxes, including beeswax, glycowax, castor wax, carnauba wax, paraffins and candelilla wax), ion-exchange resins,water-insoluble proteins (e.g., zein), wicking agents (e.g., starch derivatives such as waxy maltodextrin and drum dried corn starch, cellulose derivatives such as hydroxypropylmethyl cellulose, hydroxypropyl cellulose, methyl cellulose, and carboxymethyl cellulose, alginic acid, lactose, dextrose, mannitol and talc), and surfactants.

In some embodiments, the statin coating can comprise liposomal statin particles.

If desired, one or more barrier layers can be placed over the statin coating to prevent dissolution of the statin layer prior to positioning of the catheter within the segment of the blood vessel where drug administration is intended.

Local delivery of a statin comprising cerivastatin, or a pharmaceutically acceptable salt, prodrug, clathrate, or solvate thereof can produce a distinctly different risk:benefit balance than that observed after oral administration of statin comprising cerivastatin, or a pharmaceutically acceptable salt, prodrug, clathrate, or solvate thereof.

Recent studies have demonstrated that the adverse effects of statins are a result of the metabolism of statins to the lactone form in the liver. As compared to systemic administration of the free drug, which rapids forms high levels of lactone, local delivery of a liposomal formulation of a statin comprising cerivastatin, or a pharmaceutically acceptable salt, prodrug, clathrate, or solvate thereof can alter the course of lactone formation, thereby providing a greater safety window while also affording a maximum efficacious drug level. For example, as compared to orally administered cerivastatin, local delivery of a liposomal formulation of cerivastatin to a segment of a blood vessel can provide distinct safety and efficacy advantages. Local delivery of a liposomal formulation of cerivastatin can provide for a tailored rate of release of cerivastatin from the liposomal carrier, thereby providing control of the rate of generation of toxic metabolite lactone. Local delivery of a liposomal formulation of cerivastatin can also decrease the risk of drug-drug interaction. Accordingly, local delivery of a liposomal formulation of statin comprising cerivastatin, or a pharmaceutically acceptable salt, prodrug, clathrate, or solvate thereof can provide for improved efficacy while also mitigating the risk of side effects.

### Devices

The kit according to invention comprises a drug-eluting balloon catheter configured for delivery of a statin locally and acutely to a diseased vascular segment. For example, provided herein are balloon catheters that comprise a catheter balloon that is completely or partially coated with a pharmaceutical composition comprising a statin encapsulated in lipid-based particles, wherein the statin is present in a therapeutically effective amount to treat a vascular disease in a subject in need thereof when administered locally to a diseased segment of a blood vessel in the subject via the drug-eluting balloon catheter, and wherein the statin comprises cerivastatin, or a pharmaceutically acceptable salt, prodrug, clathrate, or solvate thereof. The catheter balloon and/or the statin coating can be configured such that a therapeutically effective amount of the statin is released and/or bioavailable of when the balloon is inflated intraluminally.

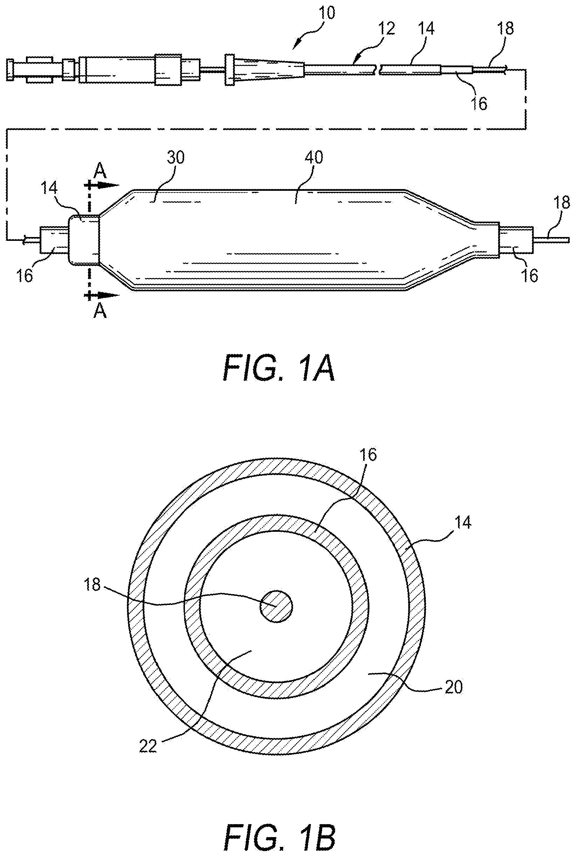

An example balloon catheter comprising a catheter balloon completely or partially coated with a statin is shown schematically in Figures 1A and 1B. As shown in Figures 1A and 1B, the balloon catheter 10 can generally include an elongated catheter shaft 12 having a proximal end and having a distal end, and an expandable balloon 30 located proximate to the distal end of the catheter shaft. The expandable balloon can have an outer surface and an inner surface disposed at the distal end portion of the catheter shaft. A coating 40 can be applied to at least one portion of the balloon catheter. In some embodiments, coating 40 can completely or partially cover the outer surface of expandable balloon 30. Coating 40 comprises a pharmaceutical composition comprising a statin encapsulated in lipid-based particles, wherein the statin is present in a therapeutically effective amount to treat a vascular disease in a subject in need thereof when administered locally to a diseased segment of a blood vessel in the subject via the drug-eluting balloon catheter, and wherein the statin comprises cerivastatin, or a pharmaceutically acceptable salt, prodrug, clathrate, or solvate thereof.

In some embodiments, the elongated catheter shaft 12 can comprise an outer tubular member 14 and an inner tubular member 16. The outer tubular member 14 can define an inflation lumen 20 that can be disposed between the proximal end portion and the distal end portion of the catheter shaft 12. Referring now to Figure 1B, the coaxial relationship between the inner tubular member 16 and the outer tubular member 14 can define an annular inflation lumen 20. The expandable member 30 is placed in fluid communication with the inflation lumen 20. The inflation lumen can supply fluid under pressure, and establish negative pressure to the expandable member. The expandable member 30 can thus be inflated and deflated. The elongated catheter can be sized and configured for delivery through a tortuous anatomy, and can further include a guidewire lumen 22 that permits it to be delivered over a guidewire 18. As illustrated in Figure 1B, the inner tubular member 16 can define the guidewire lumen 22 for the guidewire 18. Although Figures 1A and 1B illustrate the guidewire lumen as having an over-the-wire (OTW) construction, the guidewire lumen can be configured as a rapid-exchange (RX) construction, as is well known in the art.

### Kits

The embodiments of the present invention are directed to a kit according to claim 1.

The lipid-based particles can have an average particle size of from 1 nm to 200 nm (e.g., from 5 nm to 50 nm, or from 100 nm to 150 nm), as determined by dynamic light scattering.

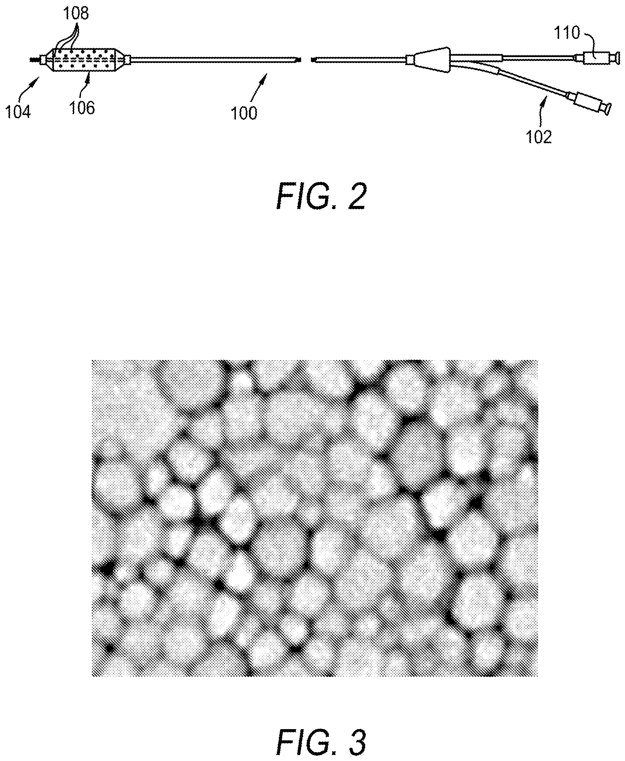

The drug-eluting balloon catheter can be any suitable drug-eluting balloon catheter discussed above. In some examples, the drug-eluting balloon catheter can comprise a porous balloon catheter. An example of a simple porous balloon catheter is schematically illustrated in Figure 2. The catheter can include an elongate flexible shaft 100 that may be formed in an extrusion process from an appropriate plastic material such as polyethylene. By way of example, when the catheter is intended to be used in the coronary arteries, the shaft may be of the order of 150 cm long and may have an outer diameter of 1.3716 mm (0.054").

. The catheter can have a proximal end 102 and a distal end 104. An inflatable and deflatable balloon, indicated generally at 106, can be mounted on the distal end 104 of the catheter shaft 100. The catheter shaft 100 can includes an inflation lumen extending from fitting 110 at the proximal end 102 to terminate at an opening within balloon 106. The inflation lumen 18 may be connected to a syringe (not shown) or other pressure fluid delivery device by way of fitting 110. Catheter shaft 10 also may be formed to include a guidewire lumen extending from the proximal end 102 of the catheter shaft to an opening at the distal end 104 of the catheter shaft. The guidewire lumen may be used to receive a guidewire by which the catheter may be guided through a subject's vasculature to the site to be treated.

Balloon 106 includes a plurality of holes or pores 108 through which medication may flow at a controlled flow rate. When the balloon 106 is inflated with a suitable pharmaceutical formulation under pressure via the inflation lumen, the formulation will seep through the holes or pores 108 in the balloon to contact the subject's vasculature. The flow rate of the pharmaceutical formulation can be controlled by the size and number of the holes or pores 108 present in the balloon 106.

By way of non-limiting illustration, examples of certain embodiments of the present disclosure are given below.

### EXAMPLES

### Preparation of Cerivastatin Formulation

A pharmaceutical formulation that included cerivastatin encapsulated in PEG-liposomes was prepared using solvent evaporation of a cerivastatin/PEG5000-DSPE mixture. Various ratios of cerivastatin:PEG5000-DSPE lipid (1:10, 1:20 and 1: 40 (w/w)) were examined. A 1:20 ratio of cerivastatin:lipid was found to suitable for the preparation of a liposomal formulation. Under these conditions, a 40-60% loading efficiency of cerivastatin in the PEG-liposomes was achieved.

The liposomal cerivastatin formulation was freeze dried using a Labconoco FREEZONE^{®} freeze dryer. The resulting liposomal particles were evaluated using a Zeiss Ultra-60 Field Emission Scanning Electron Microscope (FE-SEM). The average size of the liposomal particles, as determined using a Malvern Zetasizer Nano ZS, was 10.57 ± 0.17 nm. The zeta-potential of the liposomal particles was -13 ± 1.27 mV.

### Preparation of Liposomal Cerivastatin Formulation

Cerivastatin-loaded liposomes were prepared by thin-film hydration method. 1,2-dipalmitoyl-*sn*-glycero-3-phosphocholine (DPPC), Cholesterol, and 1,2-distearoyl-*sn*-glycero-3-phosphoethanolamine (DSPE) (59, 40, 1 mol% respectively) were used to formulate liposomes that contain cerivastatin (750 µg/ml) for *in-vitro* and *in-vivo* applications. The lipids were dissolved and mixed in an organic solvent (chloroform) in a glass flask. The lipid solutions were evaporated using rotary evaporator to form a lipid film and further dried under vacuum overnight. The lipid film was hydrated with cerivastatin-in-lOmM HEPES, 300mM sucrose, pH 7.4 buffer at 55 °C and resuspended by vigorous vortexing. The suspension was then transferred to cryogenic vials to go through 10 freeze(-196 °C)-thaw(55 °C)-vortex cycles to enhance the hydration and encapsulation processes. Then large, multilamellar vesicles (LMVs) were downsized by extrusion through two-stacks of 100nm polycarbonate filters 10 times at 55 °C to form large, unilamellar vesicles (LUVs).

### Characterization of Liposomal Cerivastatin Formulation

The particle size, size distribution, and zeta-potential analysis were evaluated using dynamic light scattering (DLS) device (Zetasizer Nano S). All measurements were conducted at 25 °C in triplicate, and were reported as Number Mean ± SD. Electron microscopy micrographs of liposomes were generated from a transmission electron microscope (TEM) Tecnai T20 operating at an acceleration voltage of 200 KV with LaB6 filament. Samples were deposited on formvar/ carbon film coated Cu 400-mesh grids and negatively stained with 2 % uranyl acetate to enhance the visualization.

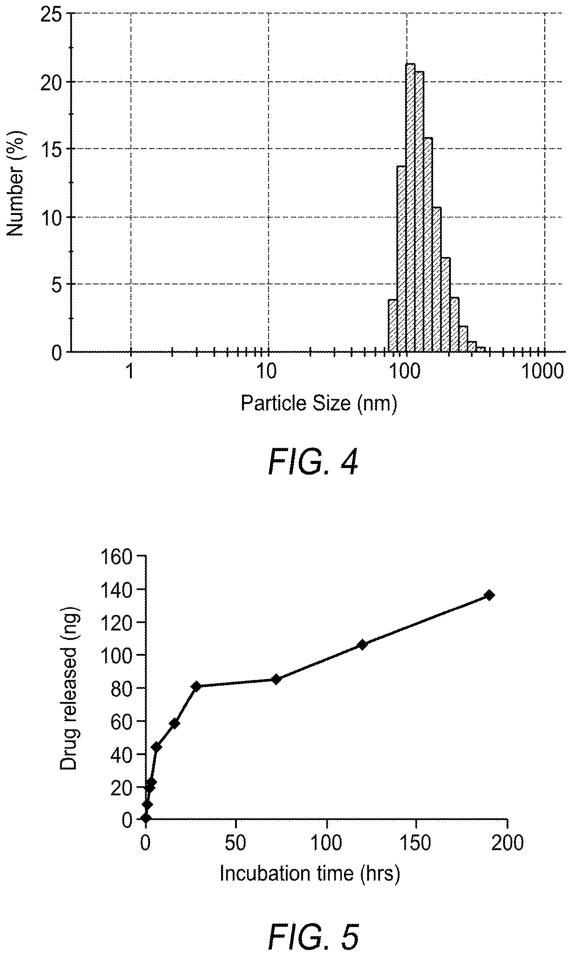

Figure 3 is a transmission electron microscopy micrograph illustrating a population of cerivastatin-loaded liposomes prepared using the methods described above. Figure 4 is a plot of the particle size distribution of cerivastatin-loaded liposomes prepared using the methods described above. As shown in Figures 3 and 4, the cerivastatin-loaded liposomes were well formed and exhibited average particle sizes of 133 nm ± 43 nm.

Release kinetic studies were performed using a dialysis method (Slide-A-Lyzer dialysis cassettes, 3500 MWCO) against 10 mM HEPES, 300 mM sucrose, pH 7.4 buffer at room temperature. The release medium was sampled at various intervals over 200 hours and refilled with fresh medium. The concentration of released cerivastatin in the medium was measured using LC-MS/MS (Agilent 6460 Triple Quad LC/MS) with Zorbax XDB C-18 analytical column (3.5 µm, 2.1 x 50 mm) at a flow rate of 0.3 mL/min over 10 min gradient. Figure 5 is a plot of the amount of cerivastatin (in ng) released from cerivastatin-loaded liposomes as a function of time (in hours). As shown in Figure 5, cerivastatin is released from the liposomes in a controlled fashion over 200 hours.

### Biological Activity of Cerivastatin and Liposomal Cerivastatin

The effect of free cerivastatin and cerivastatin-loaded liposomes on the proliferation and viability smooth muscle cells (SMCs) was also evaluated. Human artery smooth muscle cells were seeded at 20,000 cells per well on 24-well plates (3 wells per condition) in growth medium and allowed to adhere overnight at 37 °C. The cells were washed with phosphate buffered saline (PBS) and incubated with in growth medium containing free cerivastatin or cerivastatin-loaded liposomes (0.01, 0.03, 0.1, 0.3, and 1 µM) for 48 h. Cells were washed with PBS, trypsinized, and counted using a flow cytometer (Beckman-Coulter FC500).

Cell viability was measured using LIVE/DEAD^{®} fixable green dead cell stains. Smooth muscle cells were seeded at 20,000 cells per well on 24-well plates in growth medium and allowed to adhere overnight at 37 °C. Cells were washed with PBS and incubated in growth medium containing free cerivastatin or cerivastatin-loaded liposomes (0.01, 0.03, 0.1, 0.3, and 1 µM) for 48 h. Cells were washed with PBS, trypsinized and transferred 200µL into 96-well plate. Cells were incubated with 0.5µL stain for 20 min in the dark. Analyze the fluorescence level of live/dead cells using flow cytometry (Beckman-Coulter FC500) using FL1 channel.

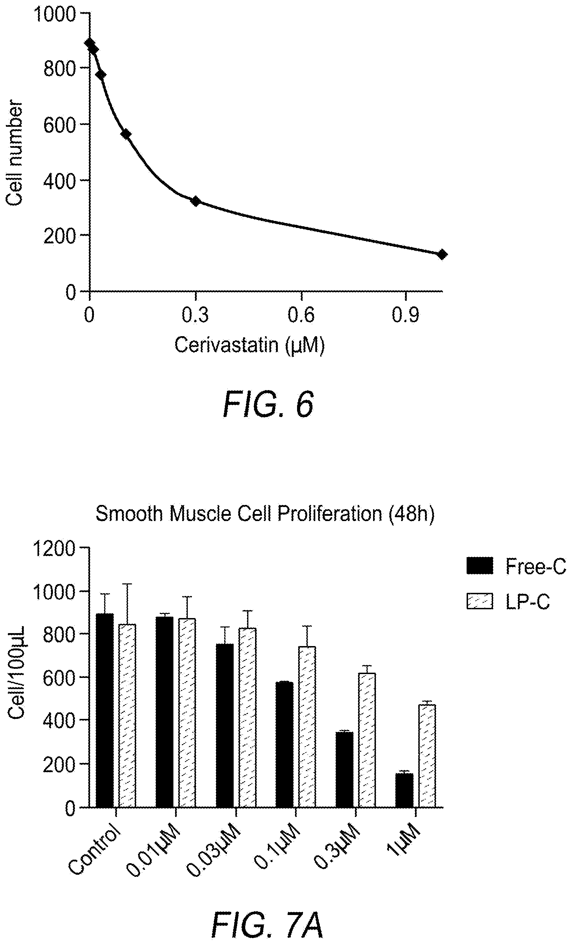

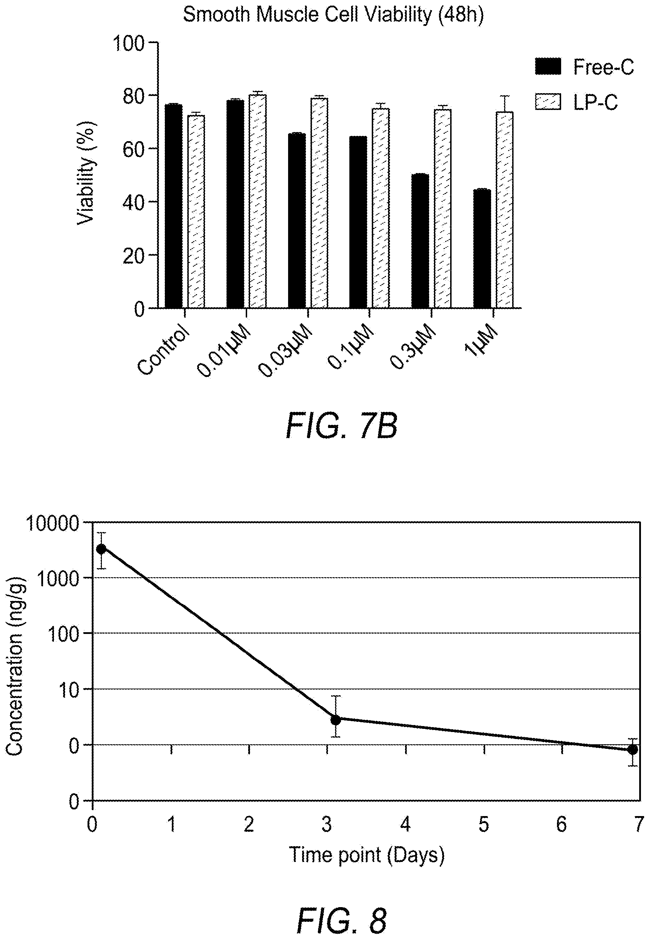

As shown in Figure 6, free cerivastatin inhibits the proliferation of SMCs. Figure 7A is a plot of the proliferation of SMCs at 48 hours following exposure to varying concentrations of free cerivastatin (black bars) and cerivastatin-loaded liposomes (grey bars). Figure 7B is a plot of the viability of SMCs at 48 hours following exposure to varying concentrations of free cerivastatin (black bars) and cerivastatin-loaded liposomes (grey bars). Surprisingly, the liposomal cerivastatin formulation inhibited SMC proliferation but did not reduce cell survival. The inhibition of cell proliferation without inducing cell toxicity by liposomal cerivastatin may imply greater safety of cerivastatin liposome formulation as compared to free cerivastatin. Lack of cell toxicity may also allow greater and accelerated endothelialization of the injured artery.

### Intraluminal Administration of Liposomal Cerivastatin Formulation

A liposomal cerivastatin formulation was intraluminally administered to selected regions of swine femoral arteries. Briefly, a weeping balloon catheter inner balloon and rapid exchange port were prepared and tracked over a 3.556 mm (0.14"). wire into the target vessel. The inner balloon was inflated to 4 atm. A liposomal cerivastatin formulation was then infused through the pores of the outer balloon. The catheter was then flushed with 0.5 ml of saline. The inner balloon pressure was maintained for additional 30 seconds. The inner balloon was then deflated and the catheter retrieved.

Section of artery receiving liposomal cerivastatin were later harvested, dissected, and flash frozen. Drug content within the arteries was determined by LC/MS/MS (quadrupole). Figure 8 is a plot of the concentration of cerivastatin (in ng) remaining within the swine artery segments following intraluminal delivery as a function of time (in days). As shown in Figure 8, the local administration of liposomal cerivastatin produces a sustained presence of cerivastatin within the arteries. This can lead to a sustained inhibition of smooth muscle cell proliferation.

1-2 mm sections of arteries were imaged using a confocal microscope at 600 nm. The results from confocal microscopy showed localization of fluorescently labeled cerivastatin liposomes in the media and adventitia of the artery. These results showed that, unlike the drug coated balloon which deposits the drug on the arterial lumen, cerivastatin liposomes delivered using the weeping balloon catheter penetrated within the arterial wall.

The term "comprising" and variations thereof as used herein is used synonymously with the term "including" and variations thereof and are open, non-limiting terms. Although the terms "comprising" and "including" have been used herein to describe various embodiments, the terms "consisting essentially of' and "consisting of' can be used in place of "comprising" and "including" to provide for more specific embodiments of the invention and are also disclosed. Other than where noted, all numbers expressing geometries, dimensions, and so forth used in the specification and claims are to be understood at the very least, and not as an attempt to limit the application of the doctrine of equivalents to the scope of the claims, to be construed in light of the number of significant digits and ordinary rounding approaches.

Unless defined otherwise, all technical and scientific terms used herein have the same meanings as commonly understood by one of skill in the art to which the disclosed invention belongs.

## Claims

1. A kit comprising a drug-eluting balloon catheter and a pharmaceutical composition comprising a statin encapsulated in lipid-based particles, wherein the statin is present in a therapeutically effective amount to treat a vascular disease in a subject in need thereof when administered locally to a diseased segment of a blood vessel in the subject via the drug-eluting balloon catheter, and wherein the statin comprises cerivastatin, or a pharmaceutically acceptable salt, prodrug, clathrate, or solvate thereof.

2. The kit of claim 1, wherein the lipid-based particles have an average particle size of from 1 nm to 200 nm, as determined by dynamic light scattering, preferably from 5 nm to 50 nm.

3. The kit of any of claims 1-2, wherein the lipid-based particles comprise liposomes or lipid micelles.

4. The kit of any of claims 1-3, wherein the drug-eluting balloon catheter comprises a porous balloon catheter.

5. The kit of any of claims 1-4, wherein the therapeutically effective amount comprises an effective amount to induce DDAH2 gene expression in vascular endothelial cells in the diseased segment of the blood vessel in the subject, an effective amount to decrease CCR2 gene expression in vascular endothelial cells in the diseased segment of the blood vessel in the subject, or a combination thereof.

6. The kit of any of claims 1-5, wherein the therapeutically effective amount comprises a dosage of from 1 ng/mm to 10 µg/mm diseased segment of the blood vessel in the subject.

7. The kit of any of claims 1-6, wherein the vascular disease is stent restenosis.

## Patentansprüche

1. Ein Set mit einem wirkstoffabgebenden Ballonkatheter und einer pharmazeutischen Zusammensetzung, die ein Statin enthält, eingekapselt in lipidbasierte Partikel, wobei das Statin in therapeutisch wirksamer Menge enthalten ist, um eine Gefäßerkrankung bei einem betroffenen Patienten zu behandeln, wenn es lokal über den wirkstoffabgebenden Ballonkatheter in den kranken Abschnitt eines Blutgefäßes verabreicht wird, und wobei das Statin Cerivastatin enthält, oder ein pharmazeutisch unbedenkliches Salz, Prodrug, Clathrat, oder ein Solvat davon.

2. Das Set nach Anspruch 1, wobei die lipid-basierten Partikel eine durchschnittliche Partikelgröße zwischen 1 nm und 200 nm haben, wie bestimmt über dynamische Lichtstreuung, vorzugsweise zwischen 5 nm und 50 nm.

3. Das Set nach irgendeinem der Ansprüche 1-2, wobei die lipid-basierten Partikel Liposome oder Lipid-Mizellen enthalten.

4. Das Set nach irgendeinem der Ansprüche 1-3, wobei der wirkstoffabgebende Ballonkatheter einen porösen Ballonkatheter umfasst.

5. Das Set nach irgendeinem der Ansprüche 1-4, wobei die therapeutisch wirksame Menge eine wirksame Menge zur Induktion der DDAH2- Genexpression in vaskulären Endothelzellen im kranken Segment des Blutgefäßes des Patienten, eine wirksame Menge zur Verringerung der CCR2 - Genexpression in vaskulären Endothelzellen im kranken Segment des Blutgefäßes des Patienten, oder eine Kombination daraus enthält.

6. Das Set nach irgendeinem der Ansprüche 1-5, wobei die therapeutisch wirksame Menge eine Dosierung zwischen 1 µg/mm und 10 µg/mm krankes Segment des Blutgefäßes des Patienten enthält.

7. Das Set nach irgendeinem der Ansprüche 1-6, wobei es sich bei der Gefäßerkrankungen um Stent-Restenosis handelt.

## Revendications

1. Kit comprenant un cathéter à ballonnet à élution médicamenteuse et une composition pharmaceutique comprenant une statine encapsulée dans des particules à base de lipides, dans lequel la statine est présente en une quantité thérapeutiquement efficace pour traiter une maladie vasculaire chez un sujet qui en a besoin lorsqu'elle est administrée localement à un segment malade d'un vaisseau sanguin du sujet via le cathéter à ballonnet à élution médicamenteuse, et dans lequel la statine comprend de la cérivastatine, ou un sel, promédicament, clathrate ou solvate pharmaceutiquement acceptable de celle-ci.

2. Kit selon la revendication 1, dans lequel les particules à base de lipides ont une taille moyenne de particules de 1 nm à 200 nm, telle que déterminée par diffusion de lumière dynamique, de préférence de 5 nm à 50 nm.

3. Kit selon l'une quelconque des revendications 1 à 2, dans lequel les particules à base de lipides comprennent des liposomes ou des micelles lipidiques.

4. Kit selon l'une quelconque des revendications 1 à 3, dans lequel le cathéter à ballonnet à élution médicamenteuse comprend un cathéter à ballonnet poreux.

5. Kit selon l'une quelconque des revendications 1 à 4, dans lequel la quantité thérapeutiquement efficace comprend une quantité efficace pour induire l'expression du gène DDAH2 dans des cellules endothéliales vasculaires dans le segment malade du vaisseau sanguin chez le sujet, une quantité efficace pour diminuer l'expression du gène CCR2 dans des cellules endothéliales vasculaires dans le segment malade du vaisseau sanguin du sujet, ou une combinaison de celles-ci.

6. Kit selon l'une quelconque des revendications 1 à 5, dans lequel la quantité thérapeutiquement efficace comprend une dose de 1 ng/mm à 10 µg/mm de segment malade du vaisseau sanguin chez le sujet.

7. Kit selon l'une quelconque des revendications 1 à 6, dans lequel la maladie vasculaire est une resténose d'endoprothèse vasculaire.
